(19) **Europäisches Patentamt / European Patent Office / Office européen des brevets**

(11) **EP 2 549 441 A1**

(12) # EUROPEAN PATENT APPLICATION
published in accordance with Art. 153(4) EPC

(43) Date of publication:
**23.01.2013 Bulletin 2013/04**

(21) Application number: **11755722.3**

(22) Date of filing: **09.03.2011**

(51) Int Cl.:
**G06T 15/00** (2011.01)   **G06T 17/00** (2006.01)
**A61B 19/00** (2006.01)

(86) International application number:
**PCT/ES2011/000069**

(87) International publication number:
**WO 2011/113972 (22.09.2011 Gazette 2011/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.03.2010 ES 201000363**

(71) Applicants:
• **Universidad Politécnica De Madrid**
  **28040 Madrid (ES)**
• **Fundacion para la Investigacion Biomedica
  Del Hospital Gregorio Marañon**
  **28007 Madrid (ES)**

(72) Inventors:
• **LEDESMA CARBAYO, María Jesús**
  **E-28040 Madrid (ES)**
• **SANTOS LLEÓ, Andrés**
  **E-28040 Madrid (ES)**

• **RUBIO GUIVERNAU, José Luis**
  **E-28040 Madrid (ES)**
• **ARENAL MAÍZ, Ángel**
  **E-28007 Madrid (ES)**
• **PÉREZ DAVID, Esther**
  **E-28007 Madrid (ES)**
• **BERMEJO THOMAS, Javier**
  **E-28007 Madrid (ES)**
• **FERNÁNDEZ AVILÉS, Francisco**
  **E-28007 Madrid (ES)**
• **DESCO MENENDEZ, Manuel**
  **28007 Madrid (ES)**

(74) Representative: **Carvajal y Urquijo, Isabel et al
Clarke, Modet & Co.
c/ Goya, 11
28001 Madrid (ES)**

(54) **METHOD FOR DISPLAYING THE INFORMATION CONTAINED IN THREE-DIMENSIONAL IMAGES OF THE HEART**

(57)   This invention describes a method, the starting point of which is a three-dimensional image of the heart. A region of interest is defined on said image: Moreover, the surface which will be represented in the display in the form of a three-dimensional polygon mesh is defined. Each vertex of the mesh is associated with a function which assigns weights to each element in the region of interest. Display parameters are assigned to the vertices using said functions together with the intensity values of the elements in the region of interest. Said parameters are used to generate the interactive display of the surface. One application of the method would be the use thereof to help to characterize the myocardial substrate of ventricular tachycardia in patients with ischemic heart disease and to guide ablation procedures for correcting said tachycardia.

Fig. 5

EP 2 549 441 A1

**Description**

Technical Sector

The invention is encompassed in the field of display tools for supporting the diagnosis or planning of surgical or therapeutic interventions. More specifically, the method described is encompassed within the interactive three-dimensional display techniques starting from three-dimensional images acquired by means of non-invasive medical imaging techniques.

Background of the Invention

Heart and circulatory system diseases cause 1.9 million deaths in the European Union. That represents approximately half of all the deaths occurring in European countries; from this group of diseases, ischemic heart disease is a main cause of death. Ischemic heart disease leads to many premature deaths and, given that health care for cardiovascular diseases is expensive and lengthy, it also constitutes a heavy economic burden in Europe.

Complications in the form of heart rhythm disorders can arise in some types of both ischemic and non-ischemic heart diseases, such as for example ventricular tachycardia (hereinafter VT), atrial tachycardia and atrial fibrillation.

Ventricular arrhythmias are the main cause of sudden death. Although the use of the implantable cardioverter defibrillator (hereinafter ICD) prevents sudden deaths, the discharges increase non-arrhythmic mortality, therefore another type of therapy such as ablation is essential in treating these patients. Ablation of arrhythmia substrate (AAS) of VT significantly reduces the incidence of discharges in patients with ICD. It can further be used prophylactically for preventing discharges.

The annual implant rate in our country is close to 80 per million inhabitants; therefore more than 3000 devices are implanted each year. Although there are no exact figures, the number of patients carrying a defibrillator is close to 20,000. Taking into account that in 80% of cases these implants are due to ischemic heart disease and that in this group 20% have at least one annual discharge, the magnitude of this problem becomes apparent. Therefore, the non-invasive identification of VT substrates can facilitate treating many patients with ischemic heart disease.

Currently, one of the main diagnostic and treatment techniques used in patients with rhythm disorders is Electro-Anatomical Mapping (hereinafter EAM), consisting of contact measuring the electrical activity of the heart at several points thereof and depicting those measurements on a three-dimensional map. Patent US5738096, for example, describes how to use invasive probes capable of measuring the electrical activity of the heart of the patient in several locations within the heart by means of direct contact while at the same time the information about the position and orientation of the probes in each of said locations is stored. It is therefore possible to generate an electro-anatomical map of the region of interest of the heart and representing it on a three-dimensional surface, as explained in greater detail in patent EP1070480. This electro-anatomical map construction technique is of great use, however constructing detailed maps implies measuring at several hundred locations within the heart of the patient, therefore such surgical interventions can last for several hours, during which time the patient is exposed to a certain dose of ionizing radiation (X-rays) due to the fluoroscopy used during the intervention, in addition to the inherent risks of any surgical intervention, even if it is minimally invasive.

The main object of the present invention is to describe a method which allows generating maps of the myocardial substrate of certain types of arrhythmias, such as VT, similar to the maps provided by EAM, but using only the information obtained from non-invasive imaging techniques.

The diagnostic usefulness of the image generated by means of Magnetic Resonance Imaging (hereinafter MRI) in identifying the myocardial substrate of some types of cardiac arrhythmias is known in the state of the art. More specifically, the images obtained by means of Delayed Enhanced MRI (hereinafter DE-MRI), in which a gadolinium-based contrast agent is administered to the patient between 10 and 15 minutes before obtaining the images, provide valuable information about the condition of the myocardium of the patient in the presence of a disease, such as after a myocardial infarction. Publications such as S. Nazarian, et al., "Magnetic Resonance Assessment of the Substrate for Inducible Ventricular Tachycardia in Nonischemic Cardiomyopathy," Circulation, 2005, vol. 112, pp. 2821-2825 and also D. Bello, et al., "Infarct morphology identifies patients with substrate for sustained ventricular tachycardia," J. Am. Coll. Cardiol., 2005, vol. 45 pp. 1104-1108, describe the use of such images for identifying the VT substrate in patients with non-ischemic and ischemic heart diseases, respectively.

There are also other recent publications such as V.Y. Reddy, et al., "Integration of Cardiac Magnetic Resonance Imaging with Three-Dimensional Electroanatomic Mapping to Guide Left Ventricular Catheter Manipulation", J. Am. Coll. Cardiol. 2004, vol. 44, pp. 2202-2213 and F.M. Bogun et al., "Delayed-Enhanced Magnetic Resonance Imaging in Nonischemic Cardiomyopathy", J. Am. Coll. Cardiol. 2009, vol. 53, pp. 1138-1145, which relate to the possibility of combining the information from the images obtained by means of MRI with the electro-anatomical maps provided by the EAM technique.

V.Y. Reddy *et al.* describe how to record three-dimensional MRI images with EAM maps, as well as the usefulness this has when using the anatomical information provided by MRI to facilitate the interpretation of the electro-anatomical maps. Similarly, patent EP1760661 describes how to combine the electro-anatomical maps with anatomical images obtained, for example, by means of MRI or Computerized Tomography (hereinafter CT).

Furthermore, both V.Y. Reddy *et al.*, for the case of patients with chronic myocardial infarction, and F.M. Bogun *et al.*, for patients with non-ischemic heart disease, discuss the possibility of manually segmenting the surface delimiting the scar tissue for later superimposing said segmentation with the EAM generated map. Given the correlation existing between the scar tissue identified in DE-MRI and the substrate of the cardiac arrhythmias, said publications explain how this identification is useful for guiding procedures of ablation of arrhythmia substrate (hereinafter AAS).

In light of the state of the art, the diagnostic relevance of images provided by ' the DE-MRI technique in identifying the substrate of some types of cardiac arrhythmias such as VT, including both those associated with ischemic and non-ischemic heart diseases is therefore clear. However, displaying all the information contained in the three-dimensional images is not easy, it is usually being done by means of representing successive two-dimensional sections each time showing sub-sets of said information. This is not the most convenient for locating the arrhythmia substrate, such as slow conduction areas surrounded by scar tissue forming the substrate of some types of VT, since it requires the specialist to integrate the information of the successive sections. Therefore, this invention proposes a method for generating a representation in the form of a three-dimensional surface with a color map compressing the information available in the entire volume, similarly to how it is shown in EAM electro-anatomical maps. Said representation has enormous diagnostic use and value and the methods described in V.Y Reddy *et al.* and F.M. Bogun *et al*, are a first step in this direction. However, a manual segmentation separating healthy tissue from scar tissue is carried out in both publications, which implies a loss of information since not all the details contained in the DE-MRI images in the form of different grey scales between the different tissues of interest are used.

In the state of the art there are methods for representing information from a three-dimensional image on a suitably colored surface. For example patent EP0961993 describes a method within the field of victual endoscopy for generating surfaces representing morphological characteristics, such as curvature, convexity and thickness, of the wall of an organ with lumen such as the colon. To that end, first the surface of interest such as an iso-surface is defined within the three-dimensional image, and then the values of the image for determining the mentioned morphological parameters are examined in the direction perpendicular to each point of said surface.

In this same line, patent EP1458292 proposes a method which can generally be applied to any hollow organ. The method proposed in that document describes how to project the information contained in a layer of predetermined thickness within the wall of the organ on the inner surface of said organ from a three-dimensional image in which the organ of interest appears. There is also another method described in patent US6181348 which is very similar to that of patent EP1458292, although it is not limited to hollow organs.

However, the methods described in these patents are not applicable to the problem inspiring the present invention, which is generating a useful display for supporting the diagnosis and planning of surgical interventions in patients with arrhythmias, such as for example VT, providing information similar to that obtained by means of the EAM technique, and at the same time preventing some of the drawbacks of said technique by using only three-dimensional images of the heart of the patient obtained non-invasively.

The main reason that said methods are not applicable to the present problem is the form in which the display parameters of the surface are generated from the information contained in the three-dimensional image. In the method described in the present invention, display parameters are assigned to each point of the surface to be represented depending on the intensity values of a set of elements of the three-dimensional image, which in the preferred implementation will be all those which are below a distance threshold with respect to said point. This achieves emulating the measurements taken by means of EAM, in which for each location the measuring process obtains a voltage value including the contribution of all the tissue in a region surrounding the probe, with less contribution upon moving further away from the region.

In EP0961993 the display represents morphological characteristics, such as curvature, convexity and thickness of the wall of the organ of interest, which are not relevant to the problem inspiring the present invention. More so, in the method' described in document EP0961993 the displayed surface coincides with the inner surface of the organ and it is further assumed that said surface will coincide with an iso-surface of the image due to the type of images used. For the case of DE-MRI images used in the preferred implementation of the present invention, that assumption is not valid because, for example, it is easy for areas of unviable myocardium which are essential for detecting the arrhythmia substrate are mistaken with the blood from inside the ventricle in terms of intensity level, therefore the segmentation requires prior knowledge of the anatomy of the heart and cannot be solely based on the intensity levels of the image.

In documents EP1458292 and US6181348, the methods for assigning display parameters to the points of the surface are variations of the projection of the elements of the image on the points of the surface, since a series of elements of the image arranged along the direction perpendicular to the surface are evaluated for each of them. As explained above, in the preferred embodiment of this invention the operation of the EAM is emulated, so it is necessary for the display parameters of a point of the surface to be based on the set of elements of the image which are at a distance from the point below a specific threshold, which clearly cannot be achieved with methods based on projecting the elements of the image on the surface such as those mentioned above.

Having described the problem inspiring the present invention as well as the state of the art related thereto, the proposed method will be described in more detail.

Description of the Invention

The present invention describes a method for generating an interactive display of a three-dimensional surface using display parameters calculated from the information contained in a region of interest within said image starting from a three-dimensional image of the heart.

The possible applications of the proposed method include its use for supporting the diagnosis in patients with cardiac arrhythmias associated with certain types of heart diseases or for aiding in guiding surgical interventions related to said arrhythmias. A specific example of application would be supporting the identification of the myocardial substrate of ventricular tachycardias (hereinafter VT) in patients with ischemic heart diseases, and supporting the planning of ablation interventions intended for correcting said tachycardias. To that end, the method described allows generating maps of the myocardial substrate of said VT, similar to those provided by the Electro-Anatomical Mapping (hereinafter EAM) technique, but using only the information obtained from non-invasive three-dimensional imaging techniques.

The starting point of the method is a three-dimensional image of the heart, in which there is contrast according to a characteristic of the tissues of interest. Said image will have previously been obtained by means of any three-dimensional imaging technique, such as for example Computerized Tomography (CT) or Magnetic Resonance Imaging (MRI). The type of image and the contrast present therein can vary according to each specific application.

An expert defines the region of interest within the heart on said three-dimensional image, thus delimiting which elements of the image (hereinafter voxels from volume elements) will be taken into account during the following steps of the proposed method. The expert will base him/herself on anatomical and functional criteria for defining said region on the image, which will depend on the specific application. In the preferred embodiment, for example, in which the region of interest is the left ventricular myocardium, the expert will leave some structures of the heart outside the region, such as papillary muscles or other endocavity structures which in the DE-MRI images used in said embodiment coincide in intensity level with the myocardium which is included in the region

Then an expert defines a surface which will be that represented in the interactive display. The defined surface will once more be dependent on the specific application, and in a general case will be defined independently of the region of interest.

Once the surface is defined, and for facilitating its display, a representation thereof is generated in the form of a three-dimensional polygon mesh, where any of the methods known in the state of the art for generating said polygon mesh can be used. The generated mesh will be formed by vertices and arcs connecting said vertices.

The next step consists of defining a function which assigns weights to the contributions each of the voxels of the region of interest will have for each of the vertices of the mesh when calculating the statistical moments which will be used for determining the display parameters assigned to said vertex. Said function will be as follows:

$$w_i : \Omega \longrightarrow \mathbb{R}^+$$

$$i = 1 \ldots N_p \ ; \ \Omega = \{v_j : j = 1 \ldots N_v\}$$

where $N_p$ is the number of vertices of the polygon mesh and $N_v$ is the number of voxels $v_j$ within the region of interest $\Omega$ Assigning a weight equal to zero $w_i(v_j) = 0$ means that the voxel $v_j$ does not contribute to calculating display parameters for the vertex $p_i$. This weight function allows the contributions of the voxels of the region of interest to each vertex to emulate the manner in which the measurements are taken with the EAM technique. As mentioned above, in the measurements taken by means of EAM, the result obtained in each location includes contributions of all the tissue in several millimeters around the probe, but due to the type of measurement said contribution is less as the distance from the tissue to the probe increases. This can be emulated by means of a suitable definition of the weight function.

One or more statistical moments associated with each vertex are calculated from the weight function associated with each vertex of the mesh and the intensity values of the voxels belonging to the region of interest, such as for example the mean or the variance:

$$\mu_i = \frac{\sum_{j=1}^{N_v} w_i(v_j) \cdot I(v_j)}{\sum_{j=1}^{N_v} w_i(v_j)}$$

$$\sigma_i^2 = \frac{\sum_{j=1}^{N_v} w_i(v_j) \cdot \left(I(v_j) - \mu_i\right)^2}{\sum_{j=1}^{N_v} w_i(v_j)}$$

where $I(v_j)$ is the intensity value of the image in the voxel $v_j$, and $\mu_i$, $\sigma_i^2$ is the mean and variance weighted according to the weight function $\omega_i$, which would be assigned to the vertex $p_i$. Optionally, a statistical confidence measure thereof could optionally be calculated for each of said moments.

One or more display parameters, such as color for example, are calculated using the statistical moments assigned to each vertex and the interactive display is generated from said parameters and from the polygon mesh. Said display is interactive because the user can interact with it, for example, rotating, shifting, enlarging or reducing the display of the mesh.

Brief Description of the Drawings

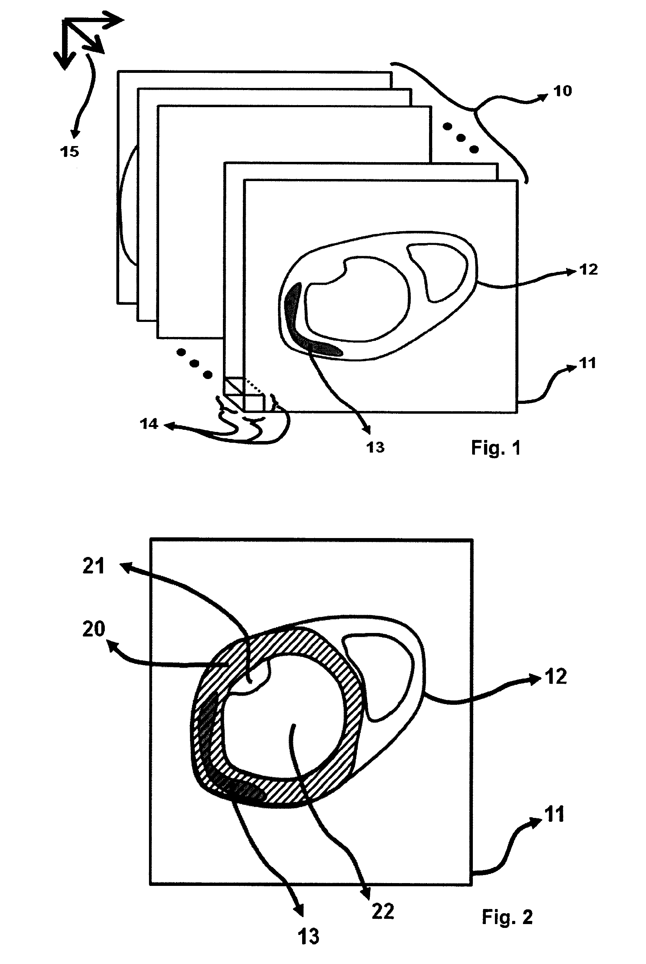

Figure 1 shows the three-dimensional image (10) divided into two-dimensional sections (11) in which the organ of interest (12) and the contrast enhancement (13) existing in some structures or tissues of interest can be distinguished. The dimensions (14) of each element or voxel of the image and the orientations (15) of the main axes thereof are also represented.

Figure 2 shows one of the two-dimensional sections (11) of the image, in which the region of interest (20) defined within the organ (12) of interest appears highlighted. Some structures (21) and (22) are also highlighted which, although they can have intensity levels similar to those of the region of interest, the expert will leave them out using prior anatomical knowledge.

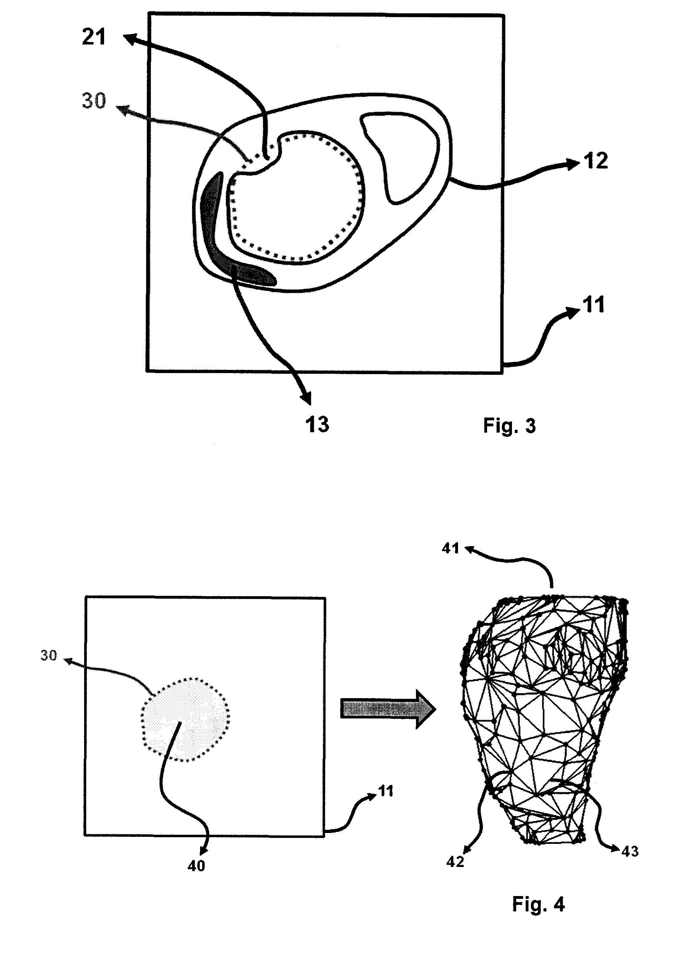

Figure 3 shows one of the two-dimensional sections (11) of the image and the intersection of the surface (30) on which the display will be generated with said two-dimensional section (11).

Figure 4, again on one of the two-dimensional sections (11), shows the mask (40) of the inside of the intersection of a section with the surface (30). The three-dimensional mesh (41) generated from the set of said masks (40), and formed by vertices (42) and by the arcs (43) connecting them, are shown in parallel

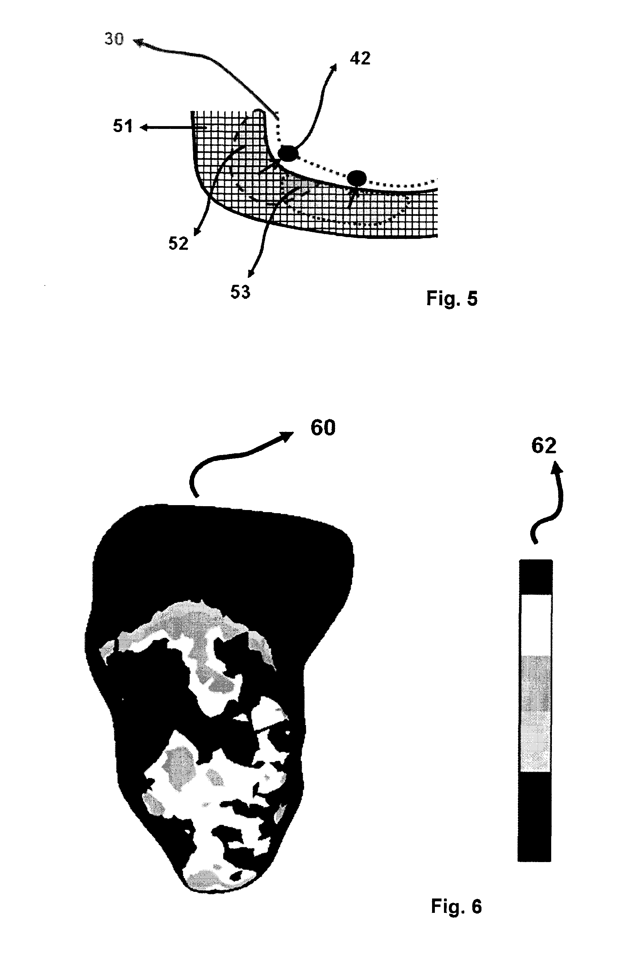

Figure 5 illustrates, within a section (11) of the image, the function which assigns weights to each of the elements or voxels (51) of the region of interest of the image. For example, the set (52) of elements or voxels contributing, by having a weight that is not zero, to said element or voxel (42) for one of the vertices (42) forming the mesh generated from the surface (30) is highlighted. It is also illustrated how there can be some elements or voxels (53) which are assigned to more than one vertex.

Figure 6 shows the display of the resulting map (60) in which colors are assigned to the different values assigned to the vertices (42) of the mesh (41) by means of a transfer function (62).

Description of a Preferred Embodiment

The present invention describes a method which generally allows obtaining an interactive display from the information contained in a region of interest within a three-dimensional image. To that end, a representation of a three-dimensional surface is generated in the form of a polygon mesh, and colors or other display parameters are assigned to the vertices of said mesh according to certain statistical moments calculated from the intensity values of the set of elements of the image (hereinafter voxels) which are within a certain region of interest.

The starting point for the preferred embodiment is a three-dimensional image of at least part of the heart of the patient obtained by means of Delayed Enhanced Magnetic Resonance Imaging (hereinafter DE-MRI). A gadolinium-based contrast agent, such as gadodiamide (Omniscan®, GE Healthcare) for example, is administered to the patient in this MRI imaging technique, and after a 10-15 minute wait an MRI image with an inversion recovery sequence is obtained. For the case of patients with an ischemic heart disease, such as those who have suffered a myocardial infarction, the images obtained by means of DE-MRI show contrast between the healthy myocardium (viable) and the myocardium scar tissue (unviable).

In the preferred embodiment, the described method is applied to images of the heart of patients with cardiac arrhythmias associated with certain types of heart diseases, such as ventricular tachycardia (hereinafter VT) for example in patients who have suffered myocardial infarction. In this embodiment, the result of the described method is an interactive display of a three-dimensional triangle mesh representing the left ventricle of the heart of the patient on which the degree of viability of the myocardial tissue in an area around each vertex of the mesh is represented according to a range of colors. Therefore by using only the information obtained by means of a non-invasive three-dimensional imaging technique such as DE-MRI, a display offering information similar to that which can be obtained by means of the Electro-Anatomical Mapping (hereinafter EAM) technique, and which is therefore useful for locating and characterizing the substrate of the VT and can therefore be used as a support for the diagnosis for these patients, as well as for planning possible therapeutic interventions intended for correcting said VT, is achieved in this preferred embodiment.

As shown in Figure 1, the starting three-dimensional image (10) can be considered as a stack of two-dimensional images (11). In the preferred embodiment, the heart of the patient (12) will appear in several of the two-dimensional sections (11) forming the image (10), and there will be contrast between healthy tissue and scar tissue (13). Additional information about the form in which the image (10) was obtained, such as the dimensions (14) of each element or voxel of the image and the orientation (15) of the three main axes of the image with respect to the body of the patient for example, will furthermore typically be available. The image can be stored, for example, according to the Digital Imaging and Communication in Medicine (DICOM) standard, and in that case the information about the dimensions and orientation of the image will be available in the image headers.

In the preferred embodiment, the three-dimensional image of the heart is obtained such that the orientation (15) of one of the main axes of the image coincides with the main axis of the left ventricle of the heart of the patient, such that in each of the two-dimensional sections (11) perpendicular to said main axis the inner contour of the left ventricle will be approximately circular in shape.

Figure 2 shows a two-dimensional section (11) of the three-dimensional image (10) in which the organ of interest (12), the heart for example, can be observed. The region of interest (20) of the organ, from which the display parameters which will be used for generating the display of the surface will subsequently be calculated, is defined within the image. Going back to the preferred embodiment, the region of interest would be the left ventricular myocardium of the patient, and the definition of said region would be done by an expert. In an alternative embodiment, the region could be defined automatically by means of an expert system which identifies, classifies and segments the anatomical or functional regions of interest, allowing for different embodiments, a computer program being the preferred solution.

Figure 3 again shows a two-dimensional section (11) of the three-dimensional image (10) in which the organ of interest (12) can be observed. It further shows the intersection of said two-dimensional section (11) with the surface (30) which will be represented in the display. In the preferred embodiment, said surface would be that which delimits the inside of the left ventricular myocardium of the patient, and therefore the surface would coincide with the inner edge of the region of interest (20) previously defined by an expert. In alternative embodiments, the surface (30) may not coincide with any of the contours of the region of interest (20). In alternative embodiments, the surface could further be defined by means of an expert system which takes in the knowledge of the specialist and automates defining the surface, allowing for different embodiments, a computer program being the preferred solution.

In the preferred embodiment, the expert will use prior anatomical knowledge for defining the region of interest and therefore the surface, since it is not always possible to segment the left ventricular myocardium using only the information provided by the intensity levels of the voxels in DE-MRI images. One of the reasons is that when segmenting the myocardium, it is usual for some structures of the heart, such as papillary muscles (21), which coincide in intensity level with the myocardium, which is included in the region (20), to be left out. Moreover, due to the type of contrast present in the DE-MRI images, the intensity levels of the unviable areas of the myocardium can coincide with those of the blood from inside ventricle (22), which is left out of the region of interest.

The next step consists of generating a three-dimensional polygon mesh representing the previously defined surface (30), where any of the methods existing in the state of the art can be used to that end.

In the preferred embodiment, given that the intersection of the surface (30) with each of the sections (11) of the image is a closed curve, the mesh can be obtained as shown in Figure 4 by means of generating for each section a mask (40) of the inside of the surface (30), which can be achieved using mathematical morphology operations for example, to subsequently use an algorithm for generating polygon meshes from iso-surfaces, such as the known "marching cubes" or "marching tetrahedrons" for example, on the volume formed by the set of said masks (40) in all the sections (31) of the image. The result is a three-dimensional point mesh (41) formed by vertices (42) and arcs (43) connecting said vertices forming the polygon mesh. In the preferred embodiment, said polygon mesh will specifically be a triangle mesh.

The next step consists of defining for each of the vertices of the mesh (42) a function which assigns weights to the contributions that each of the voxels (51) of the region of interest (20) will have in calculating the statistical moments which will be used for determining the display parameters assigned to said vertex. Said function will be as follows:

$$w_i : \Omega \longrightarrow \mathbb{R}^+$$

$$i = 1 \dots N_p \; ; \; \Omega = \{v_j : j = 1 \dots N_v\}$$

where $N_p$ is the number of vertices (42) of the polygon mesh (41) and $N_v$ is the number of voxels $v_j$ (51) within the region of interest $\Omega$ (20). Assigning a weight equal to zero $w_i(v_j) = \mathbf{0}$ means that the voxel $v_j$ does not contribute to calculating display parameters for the vertex $p_i$.

In the preferred embodiment, the objective of the weight function is to emulate the operation of the EAM, in which each measurement in a given location includes contributions of all the tissue within an area of several millimeters around the probe. To that end, in said embodiment the weight function is defined as:

$$w_i(v_j) = \begin{cases} 1 & si \quad d(v_j, p_i) \leq u_0 \\ 0 & si \quad d(v_j, p_i) > u_0 \end{cases}$$

where $u_o$ is the distance threshold and $d(v_j, p_i)$ is the Euclidean distance between the voxel $v_j$ and the vertex $p_i$, Although any other definition of distance could be used in alternative embodiments. With this definition of the weight function, it is achieved that the set (52) of voxels the Euclidean distance of which to the vertex is below a predetermined threshold $u_o$, which will have a value of between 5 mm and 10 mm for the preferred embodiment, contributes to each vertex (42) of the mesh. The dimensions (14) of the voxels will be taken into account to calculate said distance since it is typical for them to be anisotropic in DE-MRI images. Figure 5 further shows how with this definition of the weight function there can be certain voxels that are within the distance threshold from several vertices of the mesh (53) and there can also be other voxels of the region of interest of the image which do not contribute to any vertex of the mesh.

As mentioned above, ,the result obtained in each location in the measurements taken by means of EAM includes contributions of all the tissue in several millimeters around the probe, but due to the type of measurement said contribution is less as the distance from the tissue to the probe increases. In an alternative, embodiment, this behavior could be emulated by means of defining a weight function, such as:

$$w_i(v_j) = \begin{cases} f\left(d(v_j, p_i)\right) & si \quad d(v_j, p_i) \leq u_0 \\ 0 & si \quad d(v_j, p_i) > u_0 \end{cases}$$

where $f(\cdot)$ is a decreasing function, such that the weight assigned to each voxel $v_j$ of the region of interest gradually reduces as its Euclidean distance with the vertex $p_i$ increases.

In another alternative embodiment, the weight function could be defined such that each voxel of the region of interest contributes only to the closest vertex according to a measure of distance. In this case the weight function' would define a univocal correspondence since each voxel of the region of interest would be assigned to a single vertex of the mesh, the closest one according to the measure of distance used. In this case the weight function would be as follows:

$$w_i(v_j) = \begin{cases} 1 & si \quad argmin_k \left[d(v_j, p_k)\right] = i \\ 0 & si \quad argmin_k \left[d(v_j, p_k)\right] \neq i \end{cases}$$

Then one or more statistical moments associated with each vertex are calculated from the weight function associated with each vertex (42) of the mesh and the intensity values of the voxels (51) belonging to the region of interest (20). In the preferred embodiment the statistical moments calculated are the mean and the variance weighted according to the weight function corresponding to each vertex:

$$\mu_i = \frac{\sum_{j=1}^{N_v} w_i(v_j) \cdot I(v_j)}{\sum_{j=1}^{N_v} w_i(v_j)}$$

$$\sigma_i^2 = \frac{\sum_{j=1}^{N_v} w_i(v_j) \cdot (I(v_j) - \mu_i)^2}{\sum_{j=1}^{N_v} w_i(v_j)}$$

where $I(v_j)$ is the intensity value of the image in the voxel $v_j$, and $\mu_i$, $\sigma_i^2$ is the mean and variance weighted according to the weight function $w_i$, which would be assigned to the vertex $p_i$.

In said embodiment, the mean $\mu_i$ contains information about the degree of viability of the myocardium in the area around the vertex $p_i$, whereas the variance $\sigma_i^2$ provides information about the dispersion of the values of the image in said area. Therefore both moments are relevant for generating a display that is useful for supporting the diagnosis, since the mean allows distinguishing between regions of the myocardium with completely viable tissue, completely non-viable tissue, or intermediate situations, whereas the variance provides information about the heterogeneity of the tissue.

In alternative embodiments, a type of statistical confidence measure of said moments can also be calculated. For example, the confidence interval for both moments can be calculated for a desired confidence level from the number of voxels that have contributed with a weight that is not zero to calculating the mean $\mu_i$ and the variance $\sigma_i^2$.

In order to suitably display the information contained in the statistical moments assigned to each vertex, as well as in the statistical confidence measures of said moments in some embodiments, it is necessary to convert them into one or more display parameters, which can be the colors with which each vertex of the mesh will be represented for example.

In the preferred embodiment in which the statistical moments assigned to each vertex are the mean and the variance in an area of voxels around the vertex in question, the display parameters used are colors within the HSV (Hue-Saturation-Value) color space. Each vertex is assigned a color with saturation and value of 100%, and with a hue ranging linearly from 0% (red hue) corresponding to the minimum value of $\mu_i$ for $i = 1... N_p$ and 83% (purple hue) corresponding to the maximum value of $\mu_i$ for $i = 1 .... N_p$.

Figure 6 shows the display (60) generated from the three-dimensional mesh (41) and the display parameters associated with each of its vertices. The linear transfer function (62) lineal converting the values of $\mu_i$ associated with each vertex into colors within the HSV color space can be seen as part of the display.

In an alternative embodiment, the color within the HSV color space could be assigned as display parameters, but using in the assignment to each vertex the value of both the mean $\mu_i$ and the variance $\sigma_i^2$. To that end the hue (H) of each vertex would be chosen from $\mu_i$ in the same manner as in the alternative embodiment, but the value (V) would range between 0% and 100% according to $\sigma_i^2$.

In alternative embodiments in which in addition to one or more statistical moments, a statistical confidence measure thereof has been calculated, the display parameters of each vertex could be calculated from not only the statistical moments but also from the confidence measures thereof.

The display (60) is interactive because the user can interact with it for rotating, moving, enlarging or reducing the display of the polygon mesh. The transfer function determining the colors used for displaying the values of the vertices of the mesh can also be modified in an interactive manner to thus adjust the desired contrast between the areas with information of interest.

In the preferred embodiment, the display further includes information relating to the orientation of the surface represented with respect to the body of the patient, using to that end the information available about the orientation (15) of the three main axes of the image with respect to the body of the patient.

In alternative embodiments, steps of smoothing or simplifying the mesh can be included before the display to improve its visual appearance or to reduce the number of vertices. Methods existing in the state of the art, such as that described in patent US7365745 for example, can be used to that end.

Industrial Application

As mentioned in the preceding sections, this invention can be applied in the development of display tools for supporting the diagnosis or planning of surgical or therapeutic interventions.

Specifically, this invention can be used for generating maps of the myocardial substrate of certain types of arrhythmias, such as ventricular tachycardia, from three-dimensional images obtained by means of non-invasive medical imaging techniques. Since it is a method that is based solely on information obtained non-invasively, it can be used as support in choosing therapy both in patients with heart rhythm disorders and in patients susceptible to suffering said disorders.

**Claims**

1. A method for displaying the information contained in three-dimensional images of the heart, consisting of the following steps:

   1. obtaining a three-dimensional image of at least one part of the heart by means of a non-invasive technique
   2. defining a region of interest within the three-dimensional image
   3. defining a surface on which the information will be displayed
   4. generating a three-dimensional polygon mesh representing said surface, formed by vertices and arcs connecting said vertices
   5. assigning display parameters to the vertices of the three-dimensional polygon mesh
   6. generating an interactive display from the three-dimensional mesh and from the display parameters assigned to its vertices

   **characterized in that**:

   a) the three-dimensional image has contrast according to an anatomical or functional characteristic of the tissues of interest of the heart
   b) the region of interest within the heart is defined by an expert from anatomical and functional criteria
   c) the surface on which the display is carried out is defined by an expert, independently of the region of interest
   d) a weight function indicating the contribution of each voxel of the region of interest to the mentioned vertex is defined for each vertex of the polygon mesh
   e) each vertex is associated with one or more statistical moments from the weight function associated with said vertex and from the intensity values of the voxels
   f) one or more display parameters are calculated for each vertex from the statistical moments associated with said vertex.

2. The method according to that described in claim 1, wherein the three-dimensional image mentioned in a) has contrast according to myocardial tissue viability.

3. The method according to that described in claim 1, wherein the three-dimensional image mentioned in a) is obtained by means of the magnetic resonance imaging technique.

4. The method according to that described in claim 1, wherein the region of interest mentioned in b) delimits the left ventricular myocardium of the heart.

5. The method according to that described in claim 1, wherein the surface mentioned in c) coincides with the inner contour of the region of interest defined in b).

6. The method according to that described in claim 1, wherein the surface mentioned in c) coincides with the outer contour of the region of interest defined in b).

7. The method according to that described in claim 1, wherein the expert mentioned in b) is an automated expert system implemented by means of a computer program.

8. The method according to that described in claim 1, wherein the expert mentioned in c) is an automated expert system implemented by means of a computer program.

9. The method according to that described in claim 1, wherein the weight function mentioned in d) uses a measure of distance between the vertices of the polygon mesh and the voxels of the image.

10. The method according to that described in claim 9, wherein the measure of distance used is the Euclidean distance between the vertices of the polygon mesh and the voxels of the image.

11. The method according to that described in claim 10, wherein the weight function assigns decreasing weights with the value of the Euclidean distance between the vertices of the polygon mesh and the voxels of the image.

12. The method according to that described in claim 1, wherein the statistical moments mentioned in e) include at least the mean.

13. The method according to that described in claim 1, wherein the statistical moments mentioned in e) include at least the variance.

14. The method according to that described in claim 1, wherein step e) further comprises calculating one or more statistical confidence measures of the moments mentioned in e).

15. The method according to that described in claim 14, wherein both the statistical moments and the confidence measures mentioned in claim 14 are used for calculating the display parameters mentioned in f).

16. The method according to that described in claim 1, wherein the display parameters mentioned in f) include at least the color used for representing each vertex of the mesh.

17. The method according to that described in claim 16, wherein the colors are calculated in the HSV (Hue-Saturation-Value) color space.

**Fig. 1**

**Fig. 2**

Fig. 3

Fig. 4

Fig. 5

Fig. 6

EP 2 549 441 A1

# INTERNATIONAL SEARCH REPORT

International application No.

PCT/ES2011/000069

A. CLASSIFICATION OF SUBJECT MATTER

**See extra sheet**

According to International Patent Classification (IPC) or to both national classification and IPC

B. FIELDS SEARCHED

Minimum documentation searched (classification system followed by classification symbols)
G06T, A61B

Documentation searched other than minimum documentation to the extent that such documents are included in the fields searched

Electronic data base consulted during the international search (name of data base and, where practicable, search terms used)

EPODOC, INVENES, WPI, NPL, INSPEC, BIOSIS, MEDLINE

C. DOCUMENTS CONSIDERED TO BE RELEVANT

| Category* | Citation of document, with indication, where appropriate, of the relevant passages | Relevant to claim No. |
|---|---|---|
| A | DE 102006013476 A1 (Siemens AG) 04.10.2007Todo the document | 1 - 17 |
| A | DE 10340544 A1 (Siemens AG) 31.03.2005Reivindicaciones 1 - 24 | 1 - 17 |
| A | US 20090190811 A1 (Zheng et al.) 30.07.2009Todo the document | 1 - 17 |
| A | WO 2009031081 A2 (Konink. Philips Electronics) 12.03.2009Todo the document | 1 - 17 |
| A | Tops et al. "Fusion of multislice computed tomography imaging with three-dimensional electroanatomic mapping to guide radiofrequency catheter ablation procedures", Heart Rhythm, Elsevier US, vol. 2, n° 10, October 2005, pages 1076 - 1081 | 1 - 17 |

☒ Further documents are listed in the continuation of Box C.     ☒ See patent family annex.

| | | |
|---|---|---|
| * Special categories of cited documents:<br>"A" document defining the general state of the art which is not considered to be of particular relevance.<br>"E" earlier document but published on or after the international filing date<br>"L" document which may throw doubts on priority claim(s) or which is cited to establish the publication date of another citation or other special reason (as specified)<br>"O" document referring to an oral disclosure use, exhibition, or other means.<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other documents , such combination being obvious to a person skilled in the art<br>"&" document member of the same patent family | |

| Date of the actual completion of the international search<br>14/07/2011 | Date of mailing of the international search report<br>**(26/07/2011)** |
|---|---|
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>A. Cárdenas Villar<br><br>Telephone No. 91 3495393 |

Form PCT/ISA/210 (second sheet) (July 2009)

14

## INTERNATIONAL SEARCH REPORT

| | International application No. |
|---|---|
| | PCT/ES2011/000069 |

| C (continuation). | DOCUMENTS CONSIDERED TO BE RELEVANT | |
|---|---|---|
| Category * | Citation of documents, with indication, where appropriate, of the relevant passages | Relevant to claim No. |
| A | Tops et al. "Fusion of electroanatomical activation maps and multislice computed tomography to guide ablation of a focal atrial tachycardia in a Fontan patient", Journal of Cardiovascular Electrophysiology, April 2006, vol.17, n° 4, pages 431 - 434 | 1 - 17 |
| A | Den Ujil et al. "Real-time integration of intracardiac echocardiography and multislice computed tomography to guide radiofrequency catheter ablation for atrial fibrillation", Heart Rhythm, Elsevier US, vol. 5, n° 10, October 2008, pages 1403 - 1410 | 1 - 17 |
| A | Pérez-David E et al. "Noninvasive identification of ventricular tachycardia-related conducting channels using contrast-enhanced magnetic resonance imaging in patients with chronic myocardial infarction", Journal of the American College of Cardiology, January 2011, vol.57, n° 2, pages 184 - 194 | 1 - 17 |
| A | Rubio-Guivernau J. et al. "3D visualization of myocardial substrate using Delayed Enhancement MRI for pre-planning and guidance of ablation procedures of ventricular tachycardia", Journal of Cardiovascular Magnetic Resonance, February 2011, vol. 13, n° Suppl. 1, page 56 | 1 - 17 |

Form PCT/ISA/210 (continuation of second sheet) (July 2009)

## INTERNATIONAL SEARCH REPORT

Information on patent family members

| | International application No. |
| --- | --- |
| | PCT/ES2011/000069 |

| Patent document cited in the search report | Publication date | Patent family member(s) | Publication date |
| --- | --- | --- | --- |
| DE102006013476 A | 04.10.2007 | US2008075343 A | 27.03.2008 |
| DE10340544 AB | 31.03.2005 | WO2005027765 A | 31.03.2005 |
| | | AU2004273587 A | 31.03.2005 |
| | | AU2004273587 B | 10.03.2011 |
| | | EP1659967 AB | 31.05.2006 |
| | | EP20040764298 | 19.08.2004 |
| | | BRPI0413981 A | 07.11.2006 |
| | | CN1874735 A | 06.12.2006 |
| | | CN1874735 B | 26.05.2010 |
| | | JP2007503893 A | 01.03.2007 |
| | | US2007078325 A | 05.04.2007 |
| | | EP1894536 A | 05.03.2008 |
| | | EP20070075904 | 19.08.2004 |
| | | AT389363 T | 15.04.2008 |
| | | ES2302020 T | 01.07.2008 |
| US2009190811 A | 30.07.2009 | NONE | |
| WO2009031081 A | 12.03.2009 | EP2203894 AB | 07.07.2010 |
| | | EP20080807466 | 28.08.2008 |
| | | CN101796544 A | 04.08.2010 |
| | | US2010201687 A | 12.08.2010 |
| | | JP2010537701 A | 09.12.2010 |
| | | AT500574 T | 15.03.2011 |

**INTERNATIONAL SEARCH REPORT**

| International application No. |
| --- |
| PCT/ES2011/000069 |

CLASSIFICATION OF SUBJECT MATTER

*G06T15/00* (2011.01)
*G06T17/00* (2006.01)
*A61B19/00* (2006.01)

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5738096 A
- EP 1070480 A
- EP 1760661 A
- EP 0961993 A
- EP 1458292 A
- US 6181348 B
- US 7365745 B

**Non-patent literature cited in the description**

- **S. NAZARIAN et al.** Magnetic Resonance Assessment of the Substrate for Inducible Ventricular Tachycardia in Nonischemic Cardiomyopathy. *Circulation,* 2005, vol. 112, 2821-2825
- **D. BELLO et al.** Infarct morphology identifies patients with substrate for sustained ventricular tachycardia. *J. Am. Coll. Cardiol.,* 2005, vol. 45, 1104-1108
- **V.Y. REDDY et al.** Integration of Cardiac Magnetic Resonance Imaging with Three-Dimensional Electroanatomic Mapping to Guide Left Ventricular Catheter Manipulation. *J. Am. Coll. Cardiol.,* 2004, vol. 44, 2202-2213
- **F.M. BOGUN et al.** Delayed-Enhanced Magnetic Resonance Imaging in Nonischemic Cardiomyopathy. *J. Am. Coll. Cardiol.,* 2009, vol. 53, 1138-1145